Europäisches Patentamt

European Patent Office

Office européen des brevets

(11) Veröffentlichungsnummer: **0 512 237 A2**

## EUROPÄISCHE PATENTANMELDUNG

(21) Anmeldenummer: **92105240.3**

(22) Anmeldetag: **27.03.92**

(51) Int. Cl.5: **G01N 33/00**

(30) Priorität: **03.05.91 DE 4114468**

(43) Veröffentlichungstag der Anmeldung:
**11.11.92 Patentblatt 92/46**

(84) Benannte Vertragsstaaten:
**DE FR GB IT NL**

(71) Anmelder: **Hartmann & Braun
Aktiengesellschaft
Gräfstrasse 97
W-6000 Frankfurt am Main 90(DE)**

(72) Erfinder: **Ruse, Alois
Taunusstrasse 61
W-6370 Oberursel 6(DE)**

(54) Verfahren und Vorrichtung zur Aufbereitung eines zu analysierenden Gases.

(57) Verfahren und Vorrichtung zur Aufbereitung eines zu analysierenden Gases in einem Gasgemisch, um die für die Analyse unerwünschten Bestandteile aus dem Gasgemisch abzutrennen, unter Verwendung eines Aerosolfilters. Dem Gasstrom wird ein Reagenzstoff zugemischt, welcher mit unerwünschten Bestandteilen Aerosole bildet. Der Gasstrom passiert nach Einleitung des Reagenzstoffes eine Reaktionsstrecke (4), die dem Gemisch aus Gasen und dem Reagenzstoff eine Verweilzeit zur Entstehung von Aerosolen gibt. Der Reaktionsstrecke (4) ist das Aerosolfilter (5) nachgeschaltet, welches die Aerosole aus dem Gasstrom abtrennt.

Die Erfindung bezieht sich auf ein Verfahren zur Aufbereitung eines zu analysierenden Gases in einem Gasgemisch und eine Vorrichtung zur Durchführung dieses Verfahrens.

Aus der DE-PS 37 16 350, G01N 1/28, ist ein Verfahren zur Aufbereitung eines zu analysierenden Gases bekannt, bei dem in einem Gasstrom enthaltener Wasserdampf und sonstige dampfförmige Stoffe durch Abkühlung unter dem entsprechenden Taupunkt abgeschieden werden. Um das zu analysierende Gas vollständig von Wasser zu befreien, wird das als Aerosol vorhandene Wasser durch ein Aerosolfilter entfernt, welches ein die Feuchtigkeit aufnehmendes Fasermaterial enthält. Weitere unerwünschte Bestandteile im Gasstrom wie z.B. $SO_3$, Halogenide oder dergleichen werden durch chemische Reaktionen in Opfermetallfiltern entfernt.

Aus der DE-OS 36 38 096 ist ein Verfahren und eine Vorrichtung zur Abscheidung von Aerosolen in strömenden Gasen bekannt, bei dem die Gase in einer ersten Stufe einer häufigen Ablenkung ihrer Bewegungsrichtung unterworfen sind, so daß sich die Kolloide der Dispersion zu Tropfen verbinden, die durch ihre Schwerkraft ausfallen, und in einer zweiten Stufe eine Adsorptionsstrecke passieren, welche adsorptiv und desorptiv wirksam ist. Bei der Vorrichtung ist in einem Gehäuse eine von dem mit Aerosolen beladenen Gas durchströmte Filterpackung angeordnet und eine Kammer zur Aufnahme eines adsorptiv und desorptiv auf das Aerosol wirksamen Füllmaterials vorgesehen.

In der Patentschrift DD 75 828 ist ein Verfahren zur Bestimmung von Schwefeltrioxid beschrieben, bei dem der Probengasstrom in zwei kongruente Teilströmeaufgeteilt wird. Ein Teilstrom durchströmt ein auf mindestens 900°C aufgeheiztes Kontaktrohr und der andere Teilstrom wird mit Wasserdampf durchmischt durch ein Kühlrohr geleitet. Die Schwefeldioxidgehalte beider Teilströme werden gemessen und der Schwefeltrioxidgehalt durch Bildung der Differenz beider Meßwerte bestimmt.

Der Erfindung liegt die Aufgabe zugrunde, eine Möglichkeit zur Gasaufbereitung anzugeben, die es auch mit geringem apparativem Aufwand erlaubt, ein zu analysierendes Gas von den die Analyse beeinträchtigenden Substanzen ohne chemische Reaktionen zu befreien.

Diese Aufgabe wird erfindungsgemäß durch die Maßnahmen gelöst, die im kennzeichnenden Teil der Ansprüche 1 bzw. 5 beschrieben sind. Weiterbildungen der Erfindung sind Gegenstand der Unteransprüche.

Die Erfindung macht von der Tatsache Gebrauch, daß eine Vielzahl von den Gasstrom begleitende Gase mit bestimmten Stoffen spontan Nebel oder Aerosol bilden. Hierzu gehören beispielsweise $SO_3$, HCl, Silane und Chlorsilane. Die Reagenzien können flüssige oder gasförmige Stoffe sein. Im Falle von $SO_3$ oder HCl kann Wasser als Reagenzstoff dem Gasstrom zugeführt werden. Wird dem Gasstrom ein Lösemittel für Teere zugeben, dann können die sich bildenden Aerosole aus Teertröpfchen im Aerosolfilter abgeschieden werden, ohne daß der Teer im Filter erstarrt oder verharzt. Die Aufbereitung des Gases mittels Lösemittel hat sich bewährt bei Holzschwelgas, wie es zum Räuchern von Fleischwaren benutzt wird.

Das Gemisch aus den abzutrennenden Gasen und dem zugeführten Reagenzstoff benötigt eine gewisse Verweilzeit, damit das Reagenz verdampfen und mit den betreffenden Gasen möglichst große Tröpfchen bilden kann. Diese Tröpfchen werden nach Durchlaufen einer vorgegebenen Reaktionsstrecke in einem handelsüblichen Aerosolfilter abgeschieden. Hier treten die Gase, die Aerosole und der restliche Reagenzstoff in gleicher Strömungsrichtung in das Aerosolfilter ein und strömen durch die Filterporen. Durch die Benetzung der Filterporen mit den Aerosolen und gegebenenfalls mit flüssigem Reagenzstoff tritt eine Porenverengung ein und der Abscheidungsgrad des Filters steigt.

Das Filtergehäuse füllt sich mit der abgeschiedenen Flüssigkeit. Hierdurch entsteht ein Nachwascheffekt und die Verweilzeit des zu analysierenden Gases verkürzt sich. Das Gemisch aus dem Gas, aus den abgeschiedenen Aerosolen und aus überschüssigem Reagenzstoff tritt aus dem Aerosolfilter aus. In einem dem Aerosolfilter nachgeschalteten Phasentrenner wird das zu analysierende Gas von der Flüssigkeit getrennt und mit Hilfe einer Meßgasfördereinheit dem Analysator zugeführt.

Das Aerosolfilter kann auch selbst als Phasentrenner verwendet werden, wenn das dann verschlechterte Zeitverhalten der Filterung nicht stört.

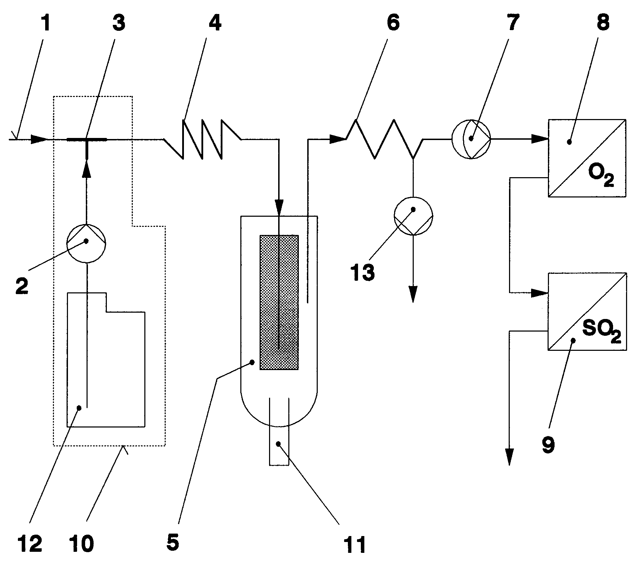

Ein Ausführungsbeispiel der Erfindung wird anhand der Zeichnung näher erläutert.

Die Figur zeigt schematisch den Gaslaufplan einer Vorrichtung zur Aufbereitung von Sauerstoff $O_2$ und Schwefeldioxid $SO_2$ in einem Gasgemisch, aus dem Schwefeltrioxid $SO_3$ als unerwünschter Bestandteil entfernt wird, indem Wasser als Reagenzstoff etwa im Verhältnis mehr als 20 Gewichtsanteile Wasser zu einem Gewichtsanteil $SO_3$ dem Gasstrom zugemischt wird.

Die Vorrichtung umfaßt eine Dosiervorrichtung 10 für die Zugabe von Wasser, eine Reaktionsstrecke 4, ein indem Gasstrom liegendes Aerosolfilter 5, einen Phasentrenner 6 und zwei Analysatoren 8 und 9 zur Bestimmung der Gaskonzentrationen.

In die Gasleitung bei 1 tritt das zu behandelnde Gasgemisch ein oder es wird durch die Saugpumpe 7 von dort angesaugt. Am T-Anschluß 3 mischt die Dosiereinrichtung 10 mit Hilfe einer Dosierpumpe 2 eine vorgegebene Menge Reaktionsstoff Wasser aus einem Gefäß 12 in das vorbeiströmende Gasgemisch. Es passiert eine Reaktionsstrecke 4, in der das $SO_3$/Wasser-Gemisch ein Aerosol bildet, bestehend aus Tröpfchen von Schwefelsäure und gebundenem Wasser. Das Gasgemisch mit dem Aerosol strömt durch das Aerosolfilter 5, in dem sich die Tröpfchen an den Filterporen abscheiden. Das Gasgemisch, überschüssiges Wasser und abgeschiedenes Aerosol verlassen gemeinsam das Aerosolfilter 5. Im nachgeschalteten Phasentrenner 6 werden die zu bestimmenden Gase, hier Sauerstoff $O_2$ und Schwefeldioxid $SO_2$, von den Flüssigkeiten getrennt, welche am Auslaß 13 ins Freie gefördert werden. Über die Saugpumpe 7 gelangen die gereinigten Gase zu den Analysatoren 8 und 9.

Die Funktion des Phasentrenners 6 kann auch das Aerosolfilter 5 übernehmen, wenn ein Abfluß 11 am Filtergehäuse vorgesehen ist, durch den die flüssigen Anteile vom Gasstrom abgeschieden werden können.

**Patentansprüche**

1. Verfahren zur Aufbereitung eines zu analysierenden Gases in einem Gasgemisch, bei dem die für die Analyse unerwünschten Bestandteile aus dem Gasgemisch abgetrennt werden, unter Verwendung eines Aerosolfilters, dadurch gekennzeichnet, daß
   - dem Gasstrom ein Reagenzstoff zugemischt wird, welcher mit unerwünschten Bestandteilen Aerosole bildet und
   - der Gasstrom nach Einleitung des Reagenzstoffes durch eine Reaktionsstrecke (4) strömt, die dem Gemisch aus Gasen und dem Reagenzstoff eine Verweilzeit zur Entstehung von Aerosolen gibt.

2. Verfahren nach Anspruch 1, dadurch gekennzeichnet, daß der Reagenzstoff (3) über eine Dosiervorrichtung (10) dem Gasstrom kontinuierlich oder getaktet zugeführt wird.

3. Verfahren nach Anspruch 1, gekennzeichnet durch Wasser als Reagenzstoff zur Beseitigung von $SO_3$ aus dem Gasstrom, wobei die Gewichtsanteile Wasser zu $SO_3$ etwa im Verhältnis größer 20 zu 1 dosiert werden.

4. Verfahren nach Anspruch 1, gekennzeichnet durch die Verwendung von Lösemitteln für Teere als Reaktionsstoff zur Beseitigung von Teeren aus dem Gasstrom.

5. Vorrichtung zur Aufbereitung eines zu analysierenden Gases in einem Gasgemisch, insbesondere zur Durchführung eines Verfahrens nach Anspruch 1, dadurch gekennzeichnet, daß der Reaktionsstrecke (4) das Aerosolfilter (5) nachgeschaltet ist, welches die Aerosole aus dem Gasstrom abtrennt.

6. Vorrichtung nach Anspruch 5, dadurch gekennzeichnet, daß das Aerosolfilter (5) mit einem Abfluß (11) versehen ist, der die Verwendung des Aerosolfilters als Phasentrenner gestattet.